(19) Europäisches Patentamt
European Patent Office
Office européen des brevets

(11) EP 0 611 762 B1

(12) EUROPÄISCHE PATENTSCHRIFT

(45) Veröffentlichungstag und Bekanntmachung des Hinweises auf die Patenterteilung:
**07.05.1997 Patentblatt 1997/19**

(51) Int Cl.6: **C07D 317/32**, C07D 303/48, C07D 307/24, C07D 317/72

(21) Anmeldenummer: **94102317.8**

(22) Anmeldetag: **16.02.1994**

(54) **Verfahren zur Herstellung von Salzen chiraler, alpha-sauerstoffunktionalisierter Carbonsäuren**

Process for preparing salts chiral carboxylic acids functionalized with an alpha-oxygen

Procédé de préparation de sels d'acides carboxyliques chirals fonctionalisé avec un alpha-oxygène

(84) Benannte Vertragsstaaten:
**DE**

(30) Priorität: **17.02.1993 DE 4304756**

(43) Veröffentlichungstag der Anmeldung:
**24.08.1994 Patentblatt 1994/34**

(73) Patentinhaber: **HOECHST AKTIENGESELLSCHAFT**
**65929 Frankfurt am Main (DE)**

(72) Erfinder:
- **Manero, Javier, Dr.**
  **D-65931 Frankfurt (DE)**
- **Leupold, Ernst Ingo, Dr.**
  **D-61267 Neu-Anspach (DE)**

(56) Entgegenhaltungen:
**EP-A- 0 189 586** **EP-A- 0 218 150**
**WO-A-90/00167**

- **Helvetica Chimica Acta,66, 814-823 (1983)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein Verfahren zur Herstellung von Salzen chiraler, α-sauerstoffunktionalisierter Carbonsäuren durch Oxidation mit Sauerstoff in Gegenwart eines metallischen Redoxkatalysators.

Chirale, α-sauerstoffunktionalisierte Carbonsäurederivate, wie Epoxy-, Dioxolan- und Tetrahydrofurancarbonsäurederivate, sind nützliche Zwischenprodukte in der organischen Synthese, beispielsweise zur Darstellung von Aminosäuren, Pheromonen und Antibiotika.
Optisch aktive Oxiranester, Tetrahydrofuran-2-carbonsäureester und 1,3-Dioxolan-4-carbonsäureester sind auch als Dotierstoffe für ferroelektrische Flüssigkristallmischungen beschrieben (z.B. EP 0 288 813, EP 0 292 954 und EP 0 355 561).

Für diese Anwendung wird eine hohe Enantiomerenreinheit gefordert, da die mit einem Enantiomeren einer bestimmten Verbindung erreichbare spontane Polarisation (siehe z.B. R.B. Meyer et al., J. de Phys. (Fr) 36, L 69 (1975)) proportional zum Enantiomerenüberschuß des betreffenden Enantiomeren ist. So ist es vorteilhaft, als Ausgangsmaterialien für die erwähnten Säurederivate die entsprechenden Alkohole oder Aldehyde zu verwenden, da sich diese aus Naturstoffen, beispielsweise durch die Spaltung von Mannit, oder durch enantioselektive Reaktionen, wie die Sharpless-Epoxidierung, in guter bis exzellenter optischer Reinheit erhalten lassen.

Um diese optische Reinheit bei der Oxidation zu den entsprechenden Carbonsäuren bzw. deren Derivaten zu erhalten, sind in der Literatur zwei Verfahren bekannt geworden.

Nach dem einen erfolgt die Oxidation mit $KMnO_4$ im wäßrigen, basischen Milieu (siehe z.B. A. Tanaka und K. Samashita, Agric. Biol. Chem. 44 (1989) 199; K. Iwadare, Bull. Chem. Soc. Jpn. 14 (1939) 131 und R. Dumont und H. Pfander, Helv. Chim. Act. 66 (1983) 814), nach dem anderen wird mit $NaIO_4$ oder $Ca(ClO)_2$ in Gegenwart eines Rutheniumsalzes in einem Gemisch aus $CCl_4$ oder $CH_3CN$ und Wasser oxidiert (siehe z.B. WO 90/00167 und K.B. Sharpless et al., J. Org. Chem. 46 (1981) 3938).

Beide Verfahren haben den Nachteil, daß bei der Aufarbeitung anorganische Übergangsmetallsalze, insbesondere die giftigen Rutheniumsalze, anfallen. Zudem werden für beide Verfahren organische Lösungsmittel, wie Ether, Acetonitril und $CCl_4$, als Reaktionsmedium oder zur Aufarbeitung und Isolierung des gewünschten Produktes benötigt. Aus ökologischer und ökonomischer Sicht war es daher wünschenswert, diese Verfahren zu verbessern. Hinzu kommt, daß die rutheniumsalz-katalysierte Reaktion direkt zu den Carbonsäuren führt, die wegen der säurelabilen Dioxolan- und Oxirangruppen nur wenig oder gar nicht lagerstabil sind.

Es wurde nun überraschend gefunden, daß die Salze chiraler, α-sauerstoffunktionalisierter Carbonsäuren ohne Verlust der optischen Reinheit durch Luftoxidation der entsprechenden Alkohole oder Aldehyde im wäßrigen Medium in Gegenwart eines metallischen Redoxkatalysators hergestellt und durch anschließende Sprühtrocknung isoliert werden können.

Die Darstellung von Carbonsäuren durch Luftoxidation ist bereits bekannt und beispielsweise in EP 0 356 703 und EP 0 502 070 beschrieben. Auch wurde eine chirale, α-sauerstoffunktionalisierte Verbindung, Saccharose, der Luftoxidation unterworfen (EP-0 218 150), es werden jedoch keine Angaben über die Enantiomerenreinheit des Endproduktes gemacht. Zudem ist diesem Dokument kein Hinweis auf eine vorteilhafte Isolierung des Reaktionsproduktes durch Sprühtrocknung zu entnehmen.

Gegenstand der Erfindung ist daher ein Verfahren zur Herstellung von Salzen chiraler, α-sauerstoffunktionalisierter Carbonsäuren der Formel (1),

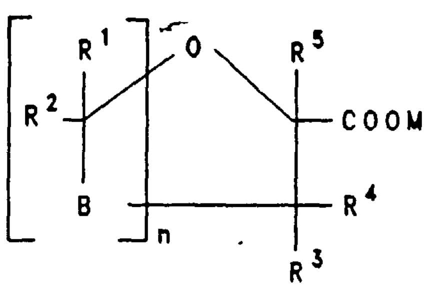

(1)

wobei die Symbole und Indizes folgende Bedeutung haben:

M : Alkali, Erdalkali;
B : -O-, $-CR^6R^7-$;
$R^1$ bis $R^7$: gleich oder verschieden H, $C_1$-$C_{16}$Alkyl, $C_2$-$C_{16}$ Alkenyl, wobei ein oder mehrere H-Atome der Alkyl- oder Alkenylgruppe durch Fluor ersetzt sein können, wobei ein oder zwei nicht benachbarte $CH_2$-Gruppen

durch -O- ersetzt sein können und wobei $R^1$ und $R^2$ zusammen $-(CH_2)_4-$, $-(CH_2)_5-$ und $(CH_2)_6-$ sein können; und

n : 0 oder 1;

dadurch gekennzeichnet, daß

a) eine Verbindung der Formel (2),

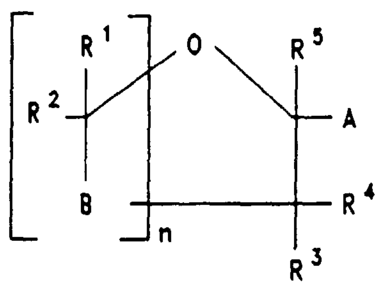

(2)

in der die Symbole und Indizes dieselbe Bedeutung wie in der Formel (1) haben und A für $-CH_2OH$ oder -CHO steht, in Wasser aufgenommen wird, so daß eine 5 bis 50 gew.-%-ige Mischung entsteht, stöchiometrische Mengen einer Base zugesetzt werden, was gegebenenfalls über den gesamten Reaktionsverlauf ausgedehnt wird, und 0,1 bis 20 Gew.-%, bezogen auf das gesamte Reaktionsgemisch eines metallischen Redoxkatalysators hinzugefügt werden,

b) durch dieses Reaktionsgemisch ein sauerstoffhaltiges Gas geleitet wird, bis der gewünschte Umsetzungsgrad erreicht ist,

c) der Katalysator abgetrennt wird und

d) die Verbindung der Formel (1) durch Sprühtrocknung isoliert wird.

Durch das erfindungsgemäße Verfahren werden die säurelabilen Carbonsäuren entsprechend der Formel (1) in hohen Ausbeuten, rein und in einer lagerstabilen Form gewonnen. In dem Verfahren werden keine organischen Lösungsmittel benötigt, und es fallen keine anorganischen Salze als Abfall an. Durch die Verwendung eines heterogenen Katalysators sind die Produkte frei von Übergangsmetallionen.

Bevorzugt werden nach dem erfindungsgemäßen Verfahren Verbindungen der Formel (1) dargestellt. Bevorzugte Verbindungen der Formel (1) sind solche, bei denen die Symbole und Indizes folgende Bedeutung haben:

M : Li, Na, K, Rb, Cs, Mg, Ca, Sr und Ba,

B : -O-, $-CH_2-$,

$R^1 = R^2$ : $CH_3$, $CF_3$ oder $R^1$ und $R^2$ zusammen sind $-(CH_2)_5-$ oder $-(CH_2)_4-$

$R^3$, $R^4$, $R^5$ : H, $C_1$-$C_{12}$-Alkyl, $C_2$-$C_{12}$-Alkenyl, wobei eine oder zwei, nicht benachbarte $CH_2$-gruppen durch -O- ersetzt sein können, und

n : 0 oder 1.

Besonders bevorzugt sind Verbindungen der Formel (1) bei denen:

M : Li, Na, K,

B : -O-, $-CH_2-$

$R^1 = R^2$ : $CH_3$ oder $R^1$ und $R^2$ zusammen $-(CH_2)_5-$;

$R^3$, $R^4$, $R^5$ : H, $C_1$-$C_9$-Alkyl, $C_2$-$C_9$-Alkenyl, mit der Maßgabe, daß stets zwei der Reste $R^3$, $R^4$ und $R^5$ H sein müssen, und

n : 0 oder 1

bedeutet.

Insbesondere bevorzugt sind die folgenden Verbindungen:

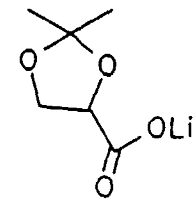

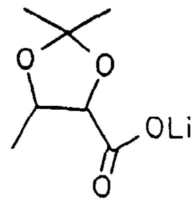

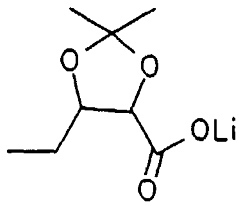

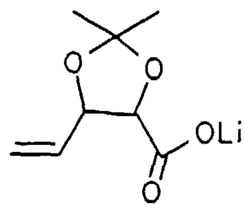

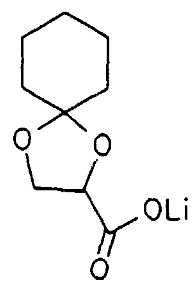

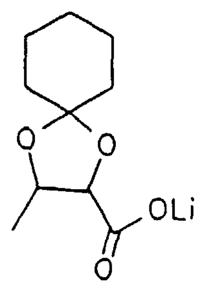

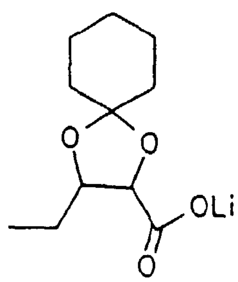

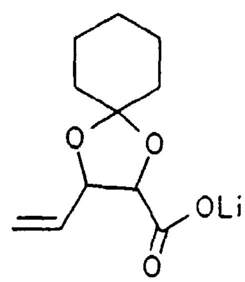

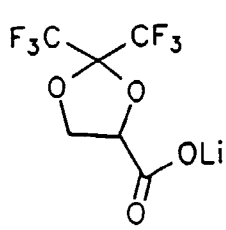

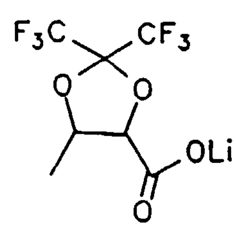

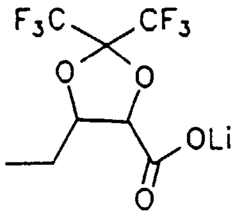
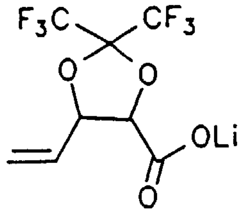

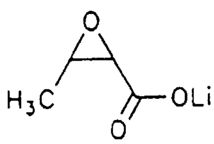
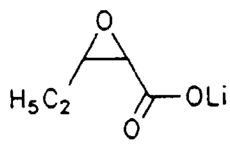

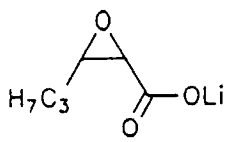
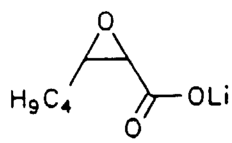

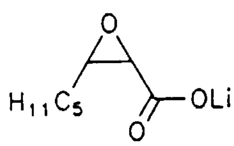
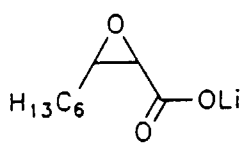

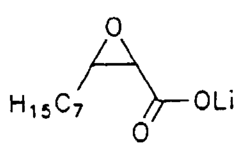
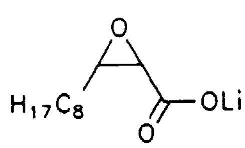

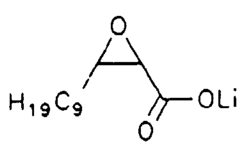

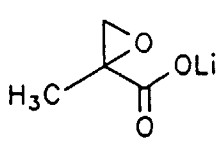
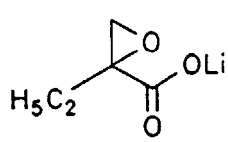

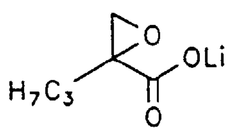

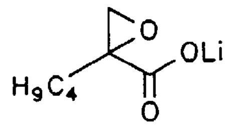

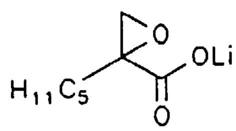

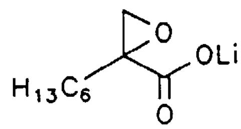

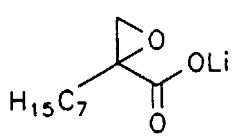

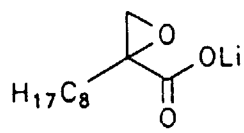

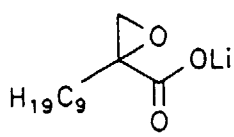

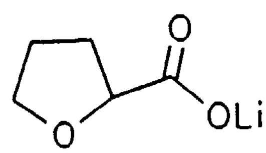

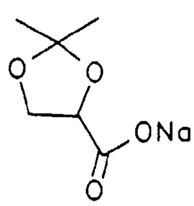

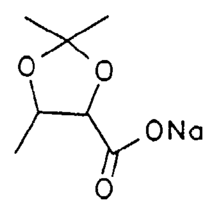

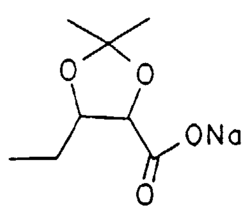

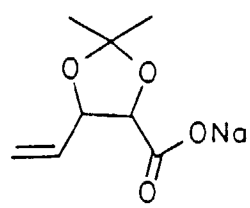

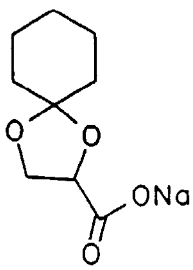

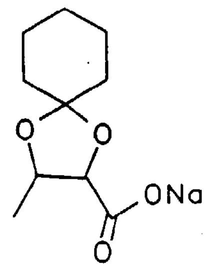

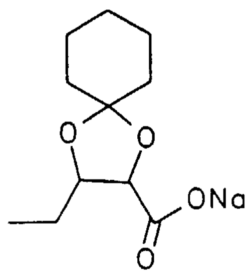

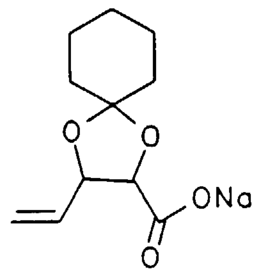

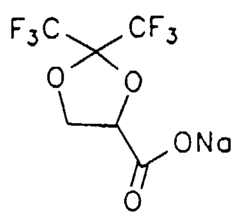

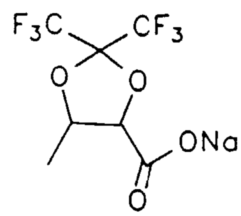

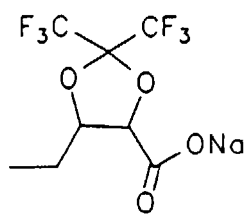

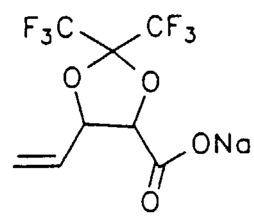

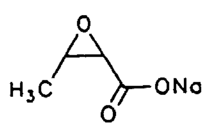

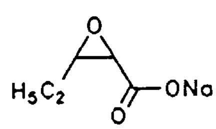

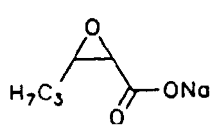

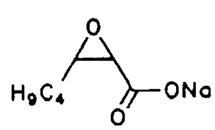

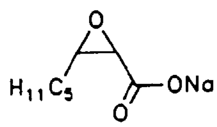
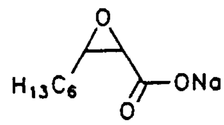

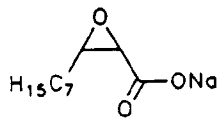
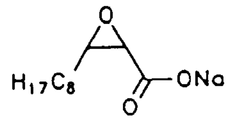

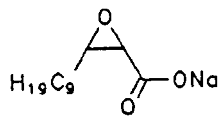

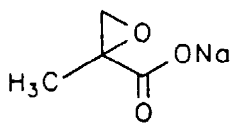
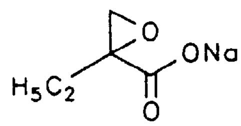

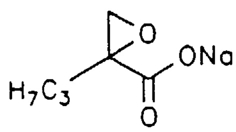
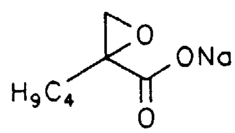

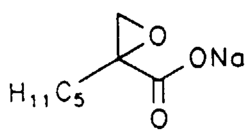
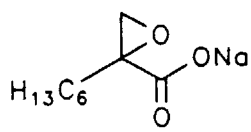

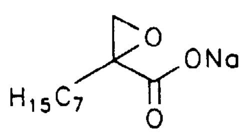
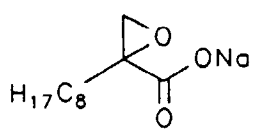

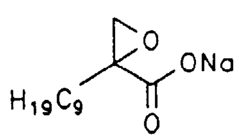

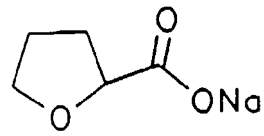

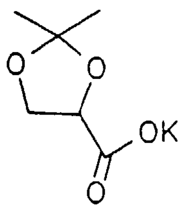

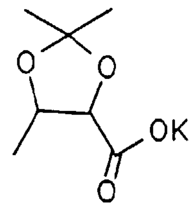

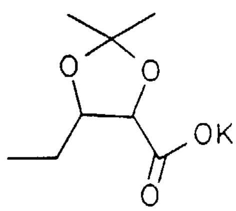

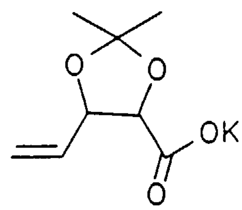

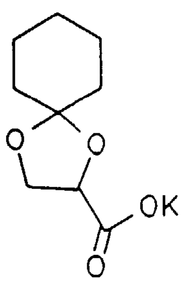

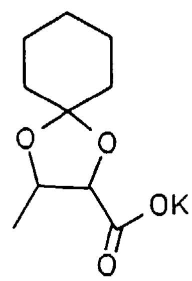

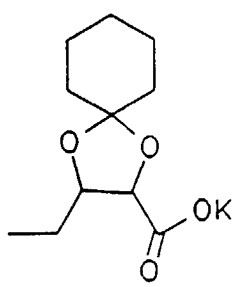

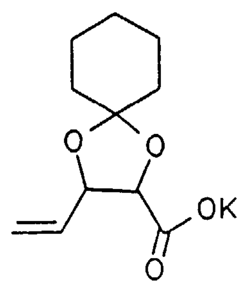

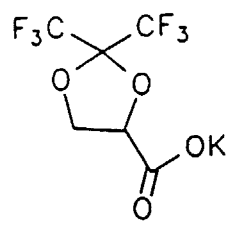
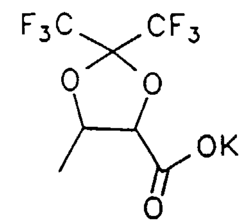

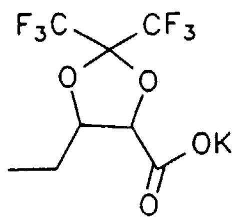
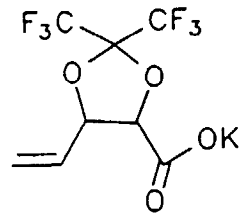

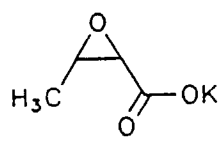
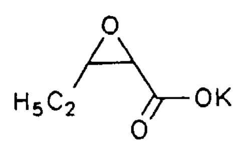

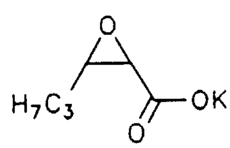
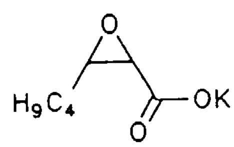

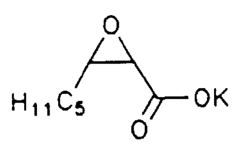
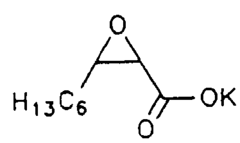

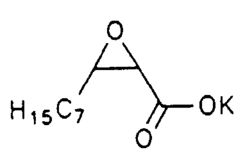
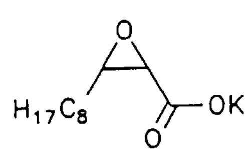

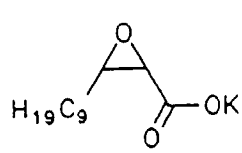

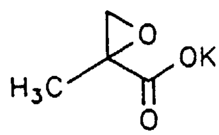
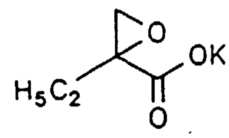

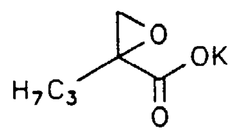
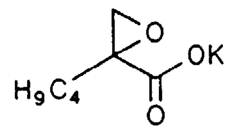

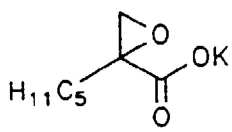
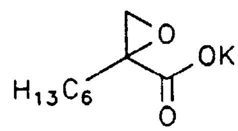

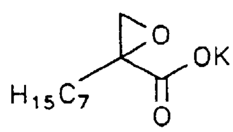
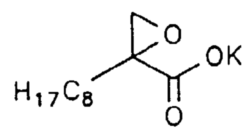

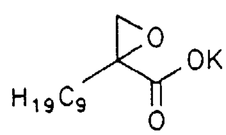

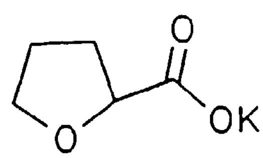

Als Ausgangsmaterialien für das erfindungsgemäße Verfahren dienen bevorzugt Verbindungen der Formel (2).
Bevorzugte Verbindungen der Formel (2) ergeben sich aus den bevorzugten Produkten der Formel (1).
Bevorzugte Ausgangsmaterialien der Formel (2) sind Alkohole (A = $-CH_2OH$) und Aldehyde (A = -CHO), besonders bevorzugt sind Alkohole. Es lassen sich aber auch alle anderen Verbindungen, die unter neutral bis basischen Bedingungen hydrolytisch zu Alkoholen oder Aldehyden gespalten werden, wie Hydrate, Halbacetale, Halbaminale und Silylether, einsetzen.
Die Ausgangsstoffe der Formel (2) sind an sich bekannt und teilweise Handelsprodukte. Es können alle Stereoisomeren (z.B. bei Oxiranen (R,R), (R,S), (S,R) und (S,S) Isomer) eingesetzt werden.
Oxirane lassen sich beispielsweise durch die Sharpless-Reaktion (siehe z.B. EP 0 046 033 und J. Am. Chem. Soc. 109 (1987) 5765.) herstellen. 1,3-Dioxolane sind teilweise käuflich erhältlich oder lassen sich beispielsweise durch Spaltung entsprechender Zuckeracetale, wie Mannitdiacetal, erhalten (siehe z.B. EP 0 288 813).
Tetrahydrofuran(THF)derivate lassen sich beispielsweise durch Hydrierung entsprechend substituierter Furanderivate herstellen.
Die Konzentration der Verbindung der Formel (2) in dem wäßrigen Medium kann in weiten Grenzen schwanken. Vorzugsweise wird (2) in einer Konzentration von 5 bis 50 Gew.-%, bezogen auf die gesamte Lösung, besonders bevorzugt

von 10 bis 40 Gew.-%, insbesondere von 15 bis 35 Gew.-%, eingesetzt.
Im Einzelfall kann es notwendig sein 5 bis 70 Gew.-% eines Lösungsvermittlers, vorzugsweise eines flüchtigen, wie Diethylenglykoldimethylether, zuzusetzen.

Als Katalysatoren eignen sich Metalle, Metallverbindungen und Legierungen, die Redoxprozesse katalysieren, vorzugsweise die Metalle der Platingruppe, wie Iridium, Rhodium, Ruthenium, besonders bevorzugt Palladium und/oder Platin. Ganz besonders bevorzugt sind Katalysatoren, die als Platinmetall nur Platin enthalten. Vorzugsweise sind die katalytisch wirksamen Substanzen auf einem Träger, insbesondere auf Aktivkohle aufgebracht. Der Gehalt des Katalysators an dem Metall, insbesondere Platin, liegt vorzugsweise bei 1 bis 10 Gew.-%. Geeignete Katalysatoren sind beispielsweise handelsübliche Katalysatoren mit 5 bis 10 Gew.-% Platin auf Aktivkohle.

Bevorzugtes Oxidationsmittel ist reiner Sauerstoff. Es können jedoch auch Mischungen von Sauerstoff mit unter den Reaktionsbedingungen inerten Gasen, z.B. in Form von Luft, beispielsweise Mischungen von Sauerstoff mit Inertgasen oder mit Luft, verwendet werden.

Im allgemeinen arbeitet man bei einem Gesamtdruck zwischen 0,5 und 100 bar. Bei steigendem Sauerstoffpartialdruck steigt die Reaktionsgeschwindigkeit deutlich an; jedoch kann hinsichtlich der Wirtschaftlichkeit des Verfahrens der Vorteil der höheren Reaktionsgeschwindigkeit durch den bei Anwendung von höherem Druck erforderlichen höheren Aufwand überkompensiert werden. Bevorzugt ist ein Druckbereich von Atmosphärendruck bis 10 bar (absolut), wobei das Arbeiten bei Atmosphärendruck besonders einfach auszuführen ist.

Das erfindungsgemäße Verfahren wird in der Regel bei einer Temperatur von 20°C bis zum Siedepunkt des wäßrigen Mediums, vorzugsweise von 20 bis 70°C, insbesondere 40 bis 60°C, durchgeführt.

Die eingesetzte Base läßt sich in breitem Rahmen variieren. Bevorzugt sind Alkali- und Erdalkalimetallhydroxide, -carbonate und Hydrogencarbonate wie Natriumhydroxid, Kaliumhydroxid, Lithiumhydroxid, Natriumcarbonat, Kaliumcarbonat, Natriumhydrogencarbonat und Kaliumhydrogencarbonat. Besonders bevorzugt sind wäßrige Lösungen von Natriumhydroxid, Kaliumhydroxid und Natriumhydrogencarbonat. Die Konzentration dieser Lösungen beträgt vorzugsweise 5 bis 50, besonders bevorzugt 10 bis 45, insbesondere 15 bis 35 Gew.-% Base.

Die Reaktion kann bezüglich des pH-Wertes unterschiedlich geführt werden. Zum Beispiel kann die stöchiometrisch berechnete Menge an Base auf einmal zugegeben werden, was insbesondere bei wenig basenempfindlichen Verbindungen, wie Tetrahydrofuranen und Dioxolanen, ein einfacheres Verfahren darstellt.

Beispielsweise ist es auch möglich, durch kontinuierliche Zugabe einer Base, wie Natriumhydroxid, Kaliumhydroxid oder entsprechender wäßriger Lösungen dieser Basen, den pH-Wert weitgehend konstant im Bereich von 6,5 bis 8, vorzugsweise 7 bis 7,5 einzustellen.

Vorzugsweise wird der pH-Wert kontinuierlich, beispielsweise mit einer Glaselektrode, gemessen, wobei die jeweils benötigte Menge an Base über einen angeschlossenen Autotitrator automatisch zugegeben wird.

Das erfindungsgemäße Verfahren verläuft in einem Dreiphasensystem aus festem Katalysator, wäßrigem Medium und gasförmigem Sauerstoff. Es kann in allen Apparaturen, die sich für die Durchführung von Reaktionen in der Flüssigphase mit oder ohne Anwendung von Überdruck eignen, durchgeführt werden. Beispiele dafür sind die Durchführung in einem Rührkessel oder in einer Blasensäule mit suspendiertem Katalysator. Die Oxidation kann aber auch als Festbettreaktion mit gekörntem Katalysator in einem Rieselphasenreaktor durchgeführt werden.

Die für die Bildung des jeweils gewünschten Reaktionsproduktes erforderliche Reaktionszeit kann zweckmäßig dadurch bestimmt werden, daß man in gewissen Zeitabständen Proben der Reaktionslösung entnimmt und analysiert. Beispielsweise kann die Ausbeute der Reaktionsprodukte auf einfache Weise durch Analyse einer Probe mit Hilfe der Hochdruckflüssigkeits-Chromatographie im Vergleich zu Standardlösungen laufend bestimmt werden. Wird die Base zutitriert, kann man durch das Ende des Basenverbrauchs auch in einfacher Weise das Reaktionsende feststellen. Die Optimierung der Reaktionszeit ist zu empfehlen, da eine unnötig verlängerte Einleitung von Sauerstoff verstärkt zu Überoxidationen, in der Folge beispielsweise zu Decarboxylierungen, und damit zum Verlust an Ausbeute bei dem gewünschten Reaktionsprodukt führen kann.

Der Katalysator kann nach bekannten Methoden beispielsweise durch Filtration oder Zentrifugieren abgetrennt werden.

Die nach Abtrennen des Katalysators gewonnene Lösung wird durch Sprühtrocknen aufgearbeitet.
Das Sprühtrocknen erfolgt nach an sich bekannten, dem Fachmann geläufigen Methoden, wie sie beispielsweise bei J.Pisecky und V. Westergard, Milchwissenschaft 26 (1971) 557 und Jakobi und Löhr, Detergents and Textile Washing, S 128 ff, VCH Verlagsgesellschaft, Weinheim 1987 beschrieben sind. In einem Trockenturm wird die Lösung über eine Zerstäubungseinrichtung (z.B. Einstoff- oder Zweistoffdüsen oder rotierende Scheiben) in einem heißem Gasstrom, z.B. von Luft oder einem inerten Gas, wie Stickstoff, zerstäubt. Die Eintrittstemperatur des Gases liegt vorzugsweise bei 70 bis 250, besonders bevorzugt bei 180°C. Gegebenenfalls kann die Sprühtrocknung mit weiteren Trockenschritten, z.B. Wirbelschichtnachtrocknung, verbunden werden. Das so gewonnene Produkt ist im allgemeinen rein genug für die direkte Weiterverarbeitung. In speziellen Fällen kann nach bekannten Verfahren, wie Chromatographie oder Umkristallisation, weiter aufgereinigt werden.

Verbindungen, die nach dem erfindungsgemäßen Verfahren hergestellt werden sind nützliche Zwischenprodukte,

beispielsweise in der Wirkstoffsynthese von Pharmaka, Kosmetika, Pflanzenschutzmitteln, Pheromonen, Aminosäuren, Verbindungen mit nichtlinearen optischen Eigenschaften und Nahrungsmittelzusätzen. Bevorzugt ist die Verwendung als Zwischenprodukte zur Herstellung von Komponenten chiraler Flüssigkristallmischungen.

Die Erfindung wird durch die Beispiele näher erläutert, ohne sie dadurch beschränken zu wollen.

Beispiel 1: (S)-(-)-2,2-Dimethyl-1,3,dioxolan-4-carbonsäurenatriumsalz

In einem Blasensäulenreaktor wird eine Lösung aus 125 g (R)-(-)-2,2-Dimethyl-4-hydroxymethyl-1,3-dioxolan und 500 ml Wasser gefüllt, man fügt 31,3 g eines handelsüblichen Platinkatalysator auf Kohleträger ( 5% Pt, Firma Degussa) zu und stellt mit einem pH-Meter und einer Perfusorpumpe mit 30% NaOH-Lösung auf einen konstanten pH-Wert von 7,5. Man leitet bei einer Temperatur von 40°C ein Sauerstoffstickstoffgemisch (Fluß: $O_2$:20l/h $N_2$:10l/h) ein, bis keine Natronlauge mehr zutitriert wird ( ~ 2h). Die Reaktionslösung wird filtriert, um den Katalysator abzutrennen, und bei 100°C sprühgetrocknet. Man erhält 80,0 g (S)-(-)-2,2-Dimethyl-1,3-dioxolan-4-carbonsäurenatriumsalz.
Ausbeute: 50%, $[\alpha]_D^{20}$ = + 41,3° (in $H_2O$).

Beispiel 2 D-α-β-Cyclohexylidenglycerinsäurenatriumsalz (D-2,2-Cyclohexyliden-1,3-dioxolan-4-carbonsäurenatriumsalz)

Aus 125 g D-α-β-Cyclohexylidenglycerol analog Beispiel 1.
T: 40 - 50°C, $O_2$:20l/h, Reaktionsdauer 5,5h Sprühtrocknung 95°C/170°C
Ausbeute 101 g = 67%, $[\alpha]_D^{20}$ = + 37,3° (in $H_2O$).

Analog wird hergestellt:

Beispiel 3 (D)-(-)-2,2-Trifluormethyl-1,3-dioxolan-4-carbonsäurenatriumsalz

Analog wird hergestellt:

Beispiel 4 D-2,2-Dimethyl-5-ethyl-1,3-dioxolan-4-carbonsäurenatriumsalz

Analog wird hergestellt:

Beispiel 5 D-2,2-Dimethyl-5-vinyl-1,3-dioxolan-4-carbonsäurenatriumsalz

Beispiel 6 (2R, 3 S)-Propyloxirancarbonsäurenatriumsalz

Aus 120 g (2S, 3S)-Propyloxiranmethanol, 1000 g $H_2O$ analog Beispiel 1.
T: 40°C $O_2$:$N_2$ 10l/h : 20l/h. Reaktionsdauer 7,5 h
pH: 7, Titration mit 8,6%-iger wäßriger $NaHCO_3$-Lösung, Sprühtrocknung 174/85°C.
Ausbeute: 96 g = 61%, $[\alpha]_D^{20}$ = -11,5°($H_2O$)c = 1.8.

Beispiel 7 Tetrahydrofuran-2-carbonsäurenatriumsalz

Aus 1 kg Tetrahydrofurfurylalkohol in 9 kg Wasser 0,5 kg Pt/C (5%) analog Beispiel 1.
T: 70°C, $O_2$:$N_2$ 215l/h : 20l/h Reaktionsdauer 10h.
Sprühtemperatur: 180°C.
Ausbeute: 992.5 g = 73%

## Patentansprüche

**1.** Verfahren zur Herstellung von Salzen chiraler, α-sauerstoffunktionalisierter Carbonsäuren der Formel (1),

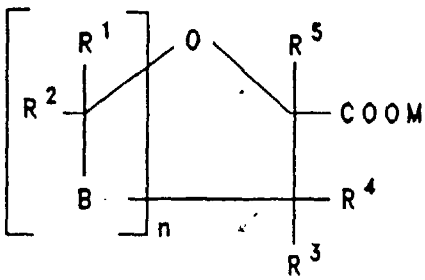

(1)

wobei die Symbole und Indizes folgende Bedeutung haben:

M : Alkali, Erdalkali;
B : -O-, $-CR^6R^7-$;
$R^1$ bis $R^7$: gleich oder verschieden H, $C_1$-$C_{16}$Alkyl, $C_2$-$C_{16}$ Alkenyl, wobei ein oder mehrere H-Atome der Alkyl- oder Alkenylgruppe durch Fluor ersetzt sein können, wobei ein oder zwei nicht benachbarte $CH_2$-Gruppen durch -O- ersetzt sein können und wobei $R^1$ und $R^2$ zusammen $-(CH_2)_4-$, $-(CH_2)_5-$ und $-(CH_2)_6-$ sein können, und
n : 0 oder 1;

dadurch gekennzeichnet, daß

a) eine Verbindung der Formel (2),

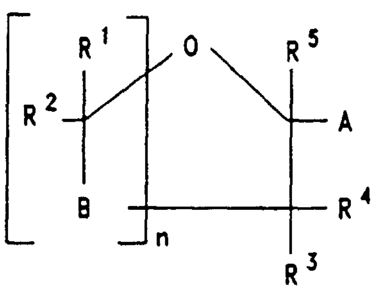

(2)

in der die Symbole und Indizes dieselbe Bedeutung wie in der Formel (1) haben und

A für $-CH_2OH$ oder -CHO steht,
in Wasser aufgenommen wird, so daß eine 5 bis 50 gew.-%-ige Mischung entsteht, stöchiometrische Mengen einer Base zugesetzt werden, was gegebenenfalls über den gesamte Reaktionsverlauf ausgedehnt wird, und 0,1 bis 20 Gew.-%, bezogen auf das gesamte Reaktionsgemisch, eines metallischen Redoxkatalysators hinzugefügt werden,

b) durch dieses Reaktionsgemisch ein sauerstoffhaltiges Gas geleitet wird, bis der gewünschte Umsetzungsgrad erreicht ist;
c) der Katalysator abgetrennt wird und
d) die Verbindung der Formel (1) durch Sprühtrocknung isoliert wird.

**2.** Verfahren nach Anspruch 1, dadurch gekennzeichnet, daß Verbindungen der Formel (2) eingesetzt werden, bei denen die Symbole und Indizes folgende Bedeutung haben:

M : Li, Na, K, Rb, Cs, Mg, Ca, Sr und Ba;
B : -O-, $-CH_2-$;
$R^1 = R^2$ : $CH_3$, $CF_3$ oder $R^1$ und $R^2$ zusammen $-(CH_2)_5-$ oder $-(CH_2)_4-$,
$R^3$, $R^4$, $R^5$ : H, $C_1$-$C_{12}$-Alkyl, $C_2$-$C_{12}$-Alkenyl, wobei eine oder zwei nicht benachbarte $CH_2$-gruppen durch -O- ersetzt sein können,

n : 0 oder 1.

3. Verfahren nach Anspruch 1, dadurch gekennzeichnet, daß der Katalysator Platin und/oder Palladium, insbesondere nur Platin enthält.

4. Verfahren nach Anspruch 1 oder 2, dadurch gekennzeichnet, daß der Katalysator aus 1 bis 10 Gew.-% mindestens eines Metalls aus der Gruppe der Platinmetalle und einem Trägermaterial, vorzugsweise Aktivkohle, besteht.

5. Verfahren nach einem oder mehreren der Ansprüche 1 bis 4, dadurch gekennzeichnet, daß man bei einem Gesamtdruck von 0,5 bis 100 bar, bevorzugt bei 1 bis 10 bar, besonders bevorzugt bei Atmosphärendruck, arbeitet.

6. Verfahren nach einem oder mehreren der Ansprüche 1 bis 5, dadurch gekennzeichnet, daß der Sauerstoff im Gemisch mit einem unter den Reaktionsbedingungen gegenüber den an der Reaktion beteiligten Verbindungen inerten Gas verwendet wird.

7. Verfahren nach einem oder mehreren der Ansprüche 1 bis 6, dadurch gekennzeichnet, daß die Umsetzung bei einer Temperatur von 30°C bis zum Siedepunkt des wäßrigen Mediums, bevorzugt bei 40 bis 95°C, besonders bevorzugt bei 50 bis 90 °C, durchgeführt wird.

8. Verfahren nach einem oder mehreren der Ansprüche 1 bis 7, dadurch gekennzeichnet, daß das Sprühtrocknen bei einer Temperatur von 70°C bis 250°C durchgeführt wird.

## Claims

1. A process for preparing salts of chiral carboxylic acids functionalized on the α-oxygen and having the formula (1)

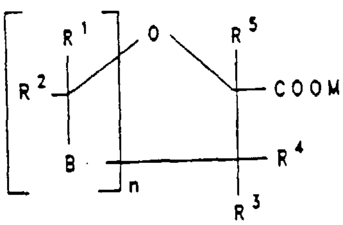

(1)

in which the symbols and indices have the following meaning:

M is an alkali metal, an alkaline earth metal;
B is -O-, $-CR^6R^7-$;
$R^1$ to $R^7$ identical or different, are H, $C_1$-$C_{16}$-alkyl, $C_2$-$C_{16}$-alkenyl, it being possible for one or more H atoms of the alkyl or alkenyl group to be replaced by fluorine, it being possible for one or two non-adjacent $CH_2$ groups to be replaced by -O-, and it being possible for $R^1$ and $R^2$ to be together $-(CH_2)_4-$, $-(CH_2)_5-$ and $-(CH_2)_6-$; and
n is 0 or 1;

which process comprises

a) taking up a compound of the formula (2),

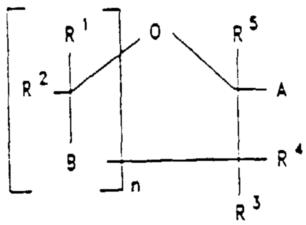

(2)

in which the symbols and indices have the same meaning as in formula (1) and

A is $-CH_2OH$ or -CHO,
in water to give a 5 to 50% by weight mixture, adding stoichiometric amounts of a base, which, if desired, is extended over the entire course of the reaction, and adding 0.1 to 20% by weight of a metallic redox catalyst, relative to the total reaction mixture,

b) passing an oxygen-containing gas through this reaction mixture until the desired degree of conversion is reached,
c) separating off the catalyst, and
d) isolating the compound of the formula (1) by spray-drying.

**2.** The process as claimed in claim 1, wherein compounds of the formula (2) are used in which the symbols and indices have the following meaning:

M is Li, Na, K, Rb, Cs, Mg, Ca, Sr and Ba;
B is -O-, $-CH_2-$;
$R^1=R^2$ is $CH_3$, $CF_3$ or $R^1$ and $R^2$ together are $-(CH_2)_5-$ or $-(CH_2)_4-$,
$R^3$, $R^4$, $R^5$ are H, $C_1$-$C_{12}$-alkyl, $C_2$-$C_{12}$-alkenyl, it being possible for one or two non-adjacent $CH_2$ groups to be replaced by -O-
n is 0 or 1.

**3.** The process as claimed in claim 1, wherein the catalyst contains platinum and/or palladium, in particular only platinum.

**4.** The process as claimed in claim 1 or 2, wherein the catalyst comprises 1 to 10% by weight of at least one platinum group metal and one support, preferably activated carbon.

**5.** The process as claimed in one or more of claims 1 to 4, wherein the reaction is carried out at a total pressure of 0.5 to 100 bar, preferably at 1 to 10 bar, particularly preferably at atmospheric pressure.

**6.** The process as claimed in one or more of claims 1 to 5, wherein the oxygen is used in a mixture with a gas which, under the reaction conditions, is inert to the compounds participating in the reaction.

**7.** The process as claimed in one or more of claims 1 to 6, wherein the reaction is carried out at a temperature of 30°C to the boiling point of the aqueous medium, preferably at 40 to 95°C, particularly preferably at 50 to 90°C.

**8.** The process as claimed in one or more of claims 1 to 7, wherein spray-drying is carried out at a temperature of 70°C to 250°C.

**Revendications**

**1.** Procédé de préparation de sels d'acides carboxyliques chiraux, à fonctionnalité oxygène en $\alpha$ de formule (I)

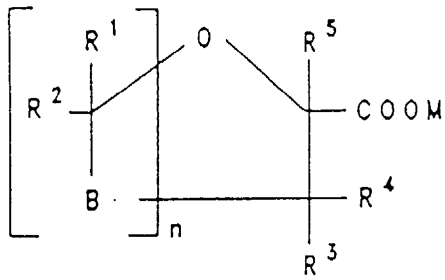

(1)

où les symboles et les indices ont les significations suivantes :

M : représente un métal alcalin, un métal alcalino-terreux ;
B : représente -O-, $-CR^6R^7-$ ;
$R^1$ à $R^7$ : identiques ou différents, représentent H, alkyle en $C_1$-$C_{16}$, alcényle en $C_2$-$C_{16}$, un ou plusieurs atome de H dans le groupe alkyle ou'alcényle pouvant être remplacés par le fluor, un ou deux groupe $CH_2$ non voisins pouvant être remplacés par -O- et $R^1$ et $R^2$ ensemble pouvant être $-(CH_2)_4-$, $-(CH_2)_5-$ et $-(CH_2)_6-$, et
n : vaut 0 ou 1

caractérisé en ce que

a) on met un composé de formule (2)

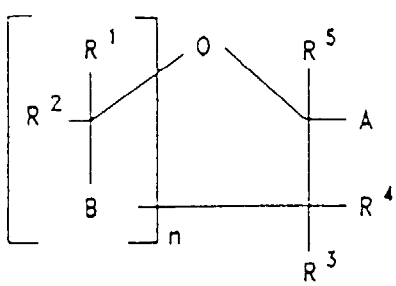

(2)

où les symboles et les indices ont la même signification que dans la formule (1), et

A représente $-CH_2OH$ ou -CHO
dans l'eau de façon telle qu'il se forme un mélange de 5 à 50 % en poids, on ajoute des quantités stoechiométriques d'une base, qui sera éventuellement augmentée au cours de l'ensemble de la réaction, et 0,1 à 20 % en poids par rapport à la totalité du mélange réactionnel, d'un catalyseur métallique redox,

b) on fait barboter un gaz contenant de l'oxygène dans le mélange réactionnel jusqu'à l'obtention du degré de réaction désiré,
c) on sépare le catalyseur et
d) on isole le composé de formule (1) par séchage par pulvérisation.

**2.** Procédé selon la revendication 1, caractérisé en ce qu'on utilise des composés de formule (2) où les symboles et les indices ont les significations suivantes :

M : représente Li, Na, K, Rb, Cs, Mg, Ca, Sr et Ba ;
B : représente -O-, $-CH_2-$ ;
$R^1 = R^2$ : représente $CH_3$, $CF_3$ ou $R^1$ et $R^2$ ensemble sont $-(CH_2)_5-$ ou $-(CH_2)_4-$,
$R^3$, $R^4$, $R^5$ : représentent H, alkyle en $C_1$-$C_{12}$, alcényle en $C_2$-$C_{12}$, un ou deux groupes $CH_2$ non voisins pouvant être remplacés par -O-,

n : vaut 0 ou 1.

3. Procédé selon la revendication 1, caractérisé en ce que le catalyseur contient du platine et/ou du palladium, plus particulièrement du platine.

4. Procédé selon la revendication 1 ou 2, caractérisé en ce que le catalyseur est composé de 1 à 10 % en poids d'au moins un métal pris dans le groupe de la famille du platine et d'un support, de préférence de charbon actif.

5. Procédé selon une ou plusieurs des revendications 1 à 4, caractérisé en ce que l'on travaille sous une pression totale de 0,5 à 100 bar, de préférence de 1 à 10 bar, de manière particulièrement préférée, à la pression atmosphérique.

6. Procédé selon une ou plusieurs des revendications 1 à 5, caractérisé en ce qu'on utilise l'oxygène en mélange avec un gaz inerte vis-à-vis des composés de réaction dans les conditions réactionnelles.

7. Procédé selon une ou plusieurs des revendications 1 à 6, caractérisé en ce qu'on met en oeuvre la réaction à une température de 30°C jusqu'au point d'ébullition du milieu aqueux, de préférence de 40 à 95°C, de manière particulièrement préférée de 50 à 90°C.

8. procédé selon une ou plusieurs des revendications 1 à 7, caractérisé en ce qu'on met en oeuvre le séchage par pulvérisation à une température de 70°C à 250°C.